# DEMANDE DE BREVET EUROPEEN

(11) **EP 1 080 692 A1**
(43) Date de publication de la demande: **07.03.2001**
(21) Numéro de dépôt: 99630066.1
(22) Date de dépôt: 03.09.1999
(51) Int. Cl.: A61B 17/70, A61B 17/64

(54) **Attache souple pour moyen d'ancrage osseux**

(71) Demandeur: Bone & Joint Research S.A., 3644 Kayl (LU)
(72) Inventeur: Michielsen, Jef, 2320 Hoogstraten (BE); Moulin, Jean, 3644 Kayl (LU); Munting, Evrard, 1390 Biez (BE); Troussel, Serge, 1470 Genappe (BE)
(74) Mandataire: Schmitz, Jean-Marie

(57) **Abrégé**

Dispositif d'attache souple destiné à solidariser un élément longitudinal (1) et un moyen d'ancrage (2) osseux, interne notamment vertébral, ou externe, ledit moyen d'ancrage (2) comportant une partie intra-osseuse et une partie filetée extra-osseuse (20) associée à un moyen de serrage (21), ledit dispositif d'attache (10) comportant une première partie de connexion (13a) et une deuxième partie de connexion (13b) destinées à recevoir la partie extra-osseuse (20) du moyen d'ancrage (2), et une partie intermédiaire (12a, 12b) réunissant lesdites première et deuxième parties de connexion (13a, 13b) destinée à enserrer l'élément longitudinal (1). Le dispositif d'attache (10) souple est constitué par un brin (11) en matériau élastique ou encore à transformation de phase, et la partie intermédiaire (12a, 12b) comprend au moins un enroulement en hélice (12a, 12b) adapté pour entourer l'élément longitudinal (1), de sorte qu'une action de rapprochement des première et deuxième parties de connexion (13a, 13b) par le moyen de serrage (21) réduit le diamètre de l'enroulement en hélice (12a, 12b) de manière à bloquer l'élément longitudinal (1) en translation et en rotation.

## Description

La présente invention concerne un dispositif d'attache souple entre un élément longitudinal et un moyen d'ancrage osseux, notamment interne ou externe.

Plus particulièrement, la présente invention est relative à un système de liaison notamment pour tige d'union générallement cylindrique comprenant un élément d'ancrage osseux comportant une partie d'ancrage intra-osseuse séparée d'une partie extra-osseuse associée à un élément de serrage. La présente invention concerne généralement un dispositif d'attache destiné notamment à relier une telle tige d'union à l'élément d'ancrage.

Dans la pratique commune, la connexion entre un moyen d'ancrage osseux et une pièce de forme globalement longitudinale est réalisée par des dispositifs divers comme des pinces articulées ou non, par des colliers ou demi-colliers, par des crochets ouverts ou fermés, par des noix d'assemblage munies de moyens de serrage divers.

Plusieurs systèmes connus permettent de bloquer la tige d'union par rapport au moyen d'ancrage osseux, mais la tige se trouve maintenue dans une orientation et ne position fixe par rapport au moyen d'ancrage. Par exemple, dans le brevet FR-A- 2 657 775 on décrit notamment un système de liaison spinal à tige destinée à relier les vertèbres comprenant une vis d'ancrage osseuse à tête filetée recevant un dispositif d'attache engagé sur la tête filetée permettant le blocage de la tige d'union entre ce dispositif d'attache et la partie conique d'un écrou de blocage engagé sur la tête filetée.

Dans le brevet US-A-5 716 355, on décrit notamment un dispositif d'attache articulé destiné à solidariser un élément longitudinal de forme essentiellement allongée et un moyen d'ancrage osseux, interne notamment vertébral, ou externe, suivant le préambule de la revendication 1. Ce dispositif d'attache comporte une première partie de connexion et une deuxième partie de connexion destinées à recevoir la partie extra-osseuse du moyen d'ancrage. En outre, ce dispositif d'attache comporte une partie intermediaire réunissant les première et deuxième parties de connexion destinée à enserrer élément longitudinal.

Ce système permet de régler dans une certaine mesure l'angle formé par les axes respectifs de la tige et du moyen d'ancrage dans un plan déterminé mais, après blocage, reste dans une position fixe, relativement rigide et ne présente pas de souplesse par effet amortisseur d'effort ou effet ressort.

Les divers dispositifs d'attache connus ont en commun les désavantages suivants:
- Absence de souplesse: pas d'effet amortisseur d'effort, pas d'effet ressort.
- Perte importante du serrage lorsque la pièce longitudinale est galbée.
- Fragilisation de la pièce longitudinale par concentration de la pression de serrage.
- Manque d'adaptabilité à une géométrie osseuse naturellement irrégulière.
- Diminution du serrage lorsque la distance entre le point d'ancrage et la pièce longitudinal croit.

Il est souhaitable de pouvoir façonner un dispositif d'attache permettant d'augmenter la variabilité d'angulation de la pièce longitudinale d'union par rapport au moyen d'ancrage tout en maintenant le serrage ou blocage des deux pièces à relier.

Les caractéristiques désirables pour un dispositif d'attache de ce type sont la variabilité d'orientation relative des deux pièces à solidariser dans toutes les directions, la possibilité d'obtenir plus de souplesse et de rappel par effet ressort; le maintien du serrage de la pièce longitudinale même lorsque l'élément est déformé, galbé longitudinalement ou présente une surface irrégulière; l'adaptabilité à une géométrie osseuse irrégulière; et le maintien du serrage lorsque la distance entre le moyen d'ancrage et la pièce longitudinale augmente.

Les dispositifs de ce genre connus antérieurement se sont révélés déficients d'une manière ou d'une autre en ce qui concerne l'obtention des caractéristiques désirées au moyen d'une structure simple, facile à monter et à appliquer.

Un but de la présente invention et de fournir un dispositif d'ancrage souple permettant d'obtenir les caractéristiques désirées mais ne nécessitant pas d'instrumentation compliquée et onéreuse.

Un autre but de la présente invention est de façonner un dispositif d'ancrage souple permettant d'obtenir une variation dans l'orientation de la pièce longitudinale d'union par rapport à l'axe du moyen d'ancrage tout en améliorant notablement le serrage des deux pièces à relier, et donc le blocage de l'ensemble.

Un autre but de l'invention est de fournir un dispositif d'ancrage présentant une souplesse axiale, radiale et en torsion tout en permettant de bloquer en translation et en rotation l'élément longitudinal même lorsque l'élément est déformé, cintré longitudinalement ou présente une surface irrégulière.

Ces buts ainsi que d'autres sont atteints, suivant l'invention, par un nouveau dispositif d'attache souple ayant les caractéristiques de la partie caractérisante de la revendication 1.

L'invention est caractérisée en ce que le dispositif d'attache souple est constitué par un brin en matériau élastique ou encore à transformation de phase, et en ce que la partie intermédiaire de ce brin comprend au moins un enroulement en hélice adapté pour entourer l'élément longitudinal, de sorte qu'une action de rapprochement des première et deuxième parties de connexion du brin par le moyen de serrage réduit le diamètre de l'enroulement en hélice de manière à bloquer l'élément longitudinal en translation et en rotation.

D'une manière générale, la présente invention qui sera décrite d'une façon plus détaillée plus loin repose sur la réduction du diamètre intérieur formé par un brin métallique enroulé en hélice à spires jointives autour d'une pièce généralement cylindrique, par la rotation relative de ses extrémités de manière à accentuer l'enroulement initial au repos.

Selon la théorie, la pression développée est proportionnelle au nombre de spires et au déplacement angulaire des extrémités.

En pratique, le frottement entre la pièce longitudinale et l'intérieur de l'hélice ainsi que le déplacement angulaire nécessaire pour obtenir le serrage croissent avec le nombre d'enroulements, conduisant à choisir de préférence leur nombre entre 1 et 3 tours. Ce principe présente l'avantage d'être tolérant envers les irrégularités de surface, ou de géométrie, telles que le cintrage ou le pliage, de la pièce longitudinale à immobiliser. Selon l'invention, le dispositif d'attache présente une souplesse axiale, radiale et en torsion tant au niveau de la partie intermédiaire que des deux parties de connexion du brin.

Un autre avantage de cet aspect de l'invention est que la souplesse axiale, radiale, et en torsion des enroulements en hélice peut être choisie selon la forme ou la grosseur du brin et est donc modulable.

Suivant un mode de réalisation avantageux de l'invention, le brin métallique en matériau élastique ou encore à transformation de phase (communément nommé métal à mémoire) est enroulé en hélice autour de l'élément longitudinal à maintenir, d'au moins un tour dans un sens, puis réalise une première boucle ou demi-boucle avant de s''enrouler en hélice de manière équivalente, de préférence symétrique, en sens inverse autour de l'élément longitudinal à maintenir.

Les extrémités dudit brin passent de part et d'autre du moyen d'ancrage et peuvent être terminées chacune par une boucle, ou de manière avantageuse réunies par une pièce coulissante, autour de la partie extra-osseuse recevant le moyen de serrage du moyen d'ancrage.

Afin d'améliorer le blocage de l'élément longitudinal tout en gardant la souplesse désirée de l'ensemble, l'action de rapprochement par le moyen de serrage réalise un déplacement angulaire de la première boucle ou demi-boucle vers les boucles ou la pièce coulissante, ce qui réduit le diamètre intérieur des enroulements en hélice de la dite attache souple autour de l'élément longitudinal de manière à le bloquer en translation et en rotation même lorsque ledit élément est déformé, cintré longitudinalement ou présente une surface irrégulière.

D'autres caractéristiques et avantages ressortiront de la description de plusieurs modes de réalisation du dispositif de l'invention. On décrira ci-après, à titre d'exemple non limitatif, une forme d'exécution préférée de la présente invention en référence aux dessins annexés dans lesquels:
- la figure 1 est une vue en perspective d'un dispositif conforme à l'invention,
- les figures 1A et 1B sont des vues en élévation latérale du dispositif suivant la figure 1 illustrant les possibilités de déplacement autour de l'axe longitudinal de la partie extra-osseuse et de coulissement sur les extrémités du brin,
- la figure 2 est une vue en perspective d'un autre dispositif conforme à l'invention, variante du dispositif de la figure 1,
- la figure 3 est une vue en perspective d'un autre dispositif conforme à l'invention,
- la figure 4 est une vue en perspective illustrant les possibilités de souplesse angulaire du dispositif de la figure 3,
- la figure 5 est une vue en perspective d'une autre dispositif conforme à l'invnetion illustrant les possibilité de souplesse axiale,
- la figure 6 est une vue en perspective d'un autre dispositif conforme à l'invention, constituée d'un brin carré,
- la figure 7 est une vue en perspective d'un autre dispositif conforme à l'invention, constituée d'un brin plat.

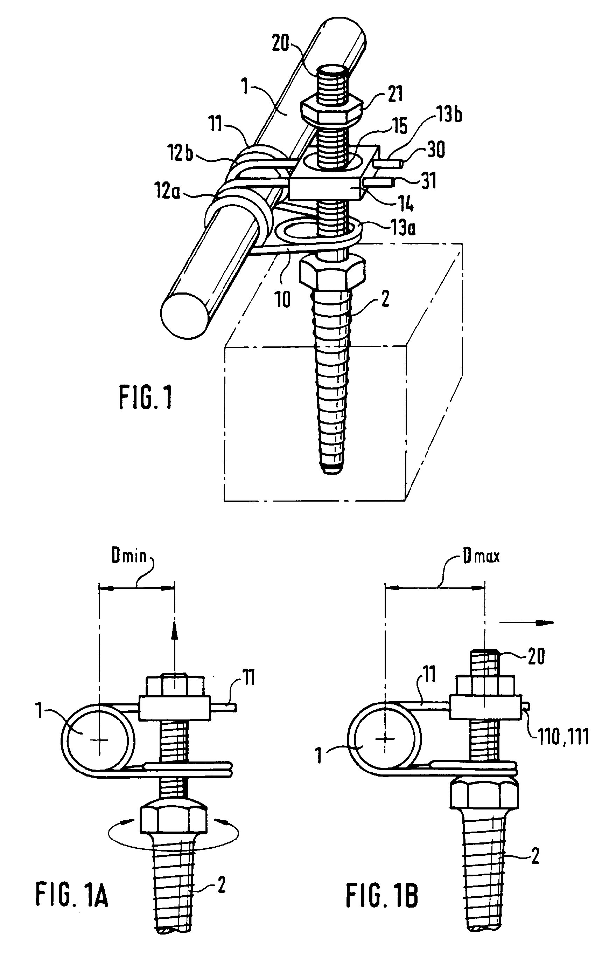

Selon la Figure 1, l'attache souple destinée à solidariser un élément longitudinal 1 et un moyen d'ancrage 2 osseux, est représentée en un mode de réalisation constitué par un brin unique métallique 11 en matériau élastique ou encore à transformation de phase (métal à mémoire), enroulé en hélices 12a et 12b autour d'un élément longitudinal 1 de forme allongée, globalement de révolution: cylindrique, avec un ou plusieurs méplats, ou ovalaire, et pouvant présenter selon la nécessité de l'application, une surface irrégulière.

Le brin 11 qui peut être de section circulaire, carrée ou rectangulaire comme un feuillard, est formé dans un matériau biocompatible de préférence métallique et réalisant par effet élastique ou mémoire, une position d'ouverture au repos permettant un coulissement de l'élément longitudinal 1.

Un premier enroulement en hélice du brin 11 est réalisé par au moins un tour dans un sens, autour de l'élément longitudinal 1, puis le brin 11 réalise une première boucle ou demi boucle 13 destinée à recevoir la partie extra-osseuse 20 du moyen d'ancrage 2, avant de s'enrouler dans un deuxiène enroulement 12b de manière équivalente, de préférence symétrique, en sens inverse autour de l'élément longitudinal 1 à maintenir.

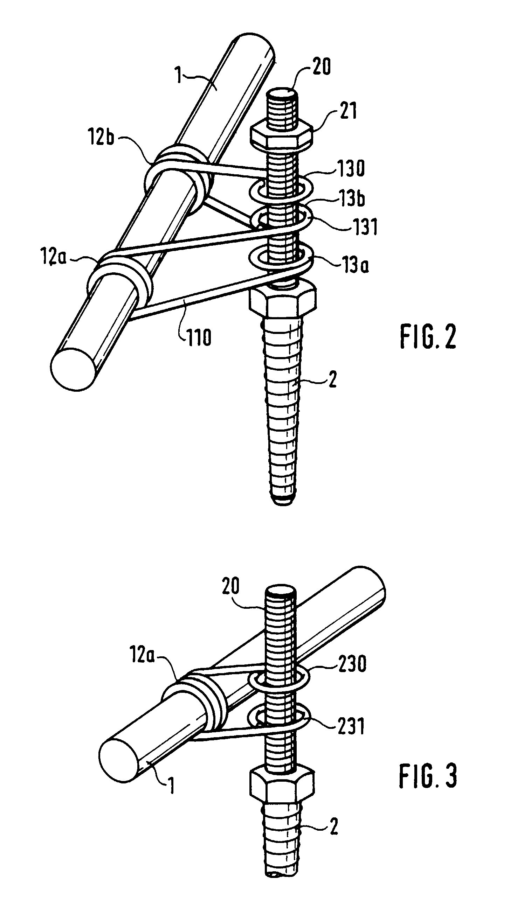

Les extrémités 30 et 31 du brin 11 passent de part et d'autre du moyen d'ancrage 2 et peuvent être terminées chacunes par une boucle 130 et 131 (fig.2), ou de manière avantageuse réunis par une pièce coulissante 14 (fig. 1) autour de la partie extra-osseuse 20 recevant le moyen de serrage 21 du moyen d'ancrage 2.

L'action de rapprochement par le moyen de serrage 21 réalise un déplacement angulaire de la première boucle ou demi-boucle 13 vers les boucles 130 et 131 ou la pièce coulissante 14, ce qui réduit le diamètre intérieur des enroulements en hélices 12a et 12b de l'attache souple 10, autour de l'élément longitudinal 1 de manière à le bloquer en translation et en rotation, même lorsque ledit élément 1 est déformé, cintré longitudinalement ou présente une surface irrégulière.

Suivant le mode de réalisation de la Figure 1, la pièce coulissante 14 est munie d'un orifice 15 destiné à recevoir la partie extra osseuse 20 et peut pivoter de selon 360 degrés en rotation, ou basculer ou se déplacer autour de l'axe longitudinal de ladite partie extra-osseuse 20. Elle peut en outre coulisser sur les extrémités 30 et 31 du brin 11 de manière à permettre un réglage de la distance entre l'élément longitudinal 1 et le moyen d'ancrage 2 osseux. L'orifice 15 destiné à recevoir la partie extra osseuse 20 est de préférence en forme de sphère concave pour s'adapter à l'embase sphérique du moyen de serrage 21 ou du moyen d'ancrage 2.

Les figures 1A et 1B illustrent les possibilités de déplacement autour de l'axe longitudinal de la partie extra-osseuse 20 et de coulissement sur les extrémités 30 et 31 du brin 11 de manière à permettre un réglage de la distance entre l'élément longitudinal 1 et le moyen d'ancrage 2 osseux par l'intermédiare de la pièce coulissante 14. La pièce coulissante 14 qui est positionnée le long des extrémités 30 et 31 du brin 11 de manière à permettre un réglage de la distance entre l'élément longitudinal 1 et le moyen d'ancrage 2 osseux, se verrouille dans la position choisie par l'action du moyen de serrage 21.

Suivant la mode de réalisation de la Figure 2, les extrémités 130 et 131 du brin 11 passent de part et d'autre du moyen d'ancrage 2 et forment chacunes une boucle 130 et 131, autour de la partie extra-osseuse 20 recevant le moyen de serrage 21 du moyen d'ancrage 2.

De manière similaire, l'action de rapprochement par le moyen de serrage 21 réalise un déplacement angulaire de la première boucle 13a vers les boucles 130 et 131, ce qui réduit le diamètre intérieur des enroulement en hélices 12a et 12b de l'attache souple 110, autour de l'élément longitudinal 1 de manière à le bloquer en translation et en rotation.

La figure 3 représente un mode de réalisation de l'attache souple 210 constitué d'un seul enroulement en hélice 12a dont le nombre de tours est compris entre un et cinq, et dont la première extrémité réalise une première boucle 230 et la seconde extrémité réalise une seconde boucle 231 destinées à recevoir la partie extra-osseuse 20 du moyen d'ancrage 2, autour duquel elles peuvent pivoter, basuler ou coulisser.

Ce mode de réalisation et particulièrement indiqué lorsque l'on desire assembler un élément longitudinal 1 et un moyen d'ancrage 2 osseux non perpendiculaires entre eux, ou lorsque l'on désire un assemblage offrant plus de souplesse et moins d'alignement.

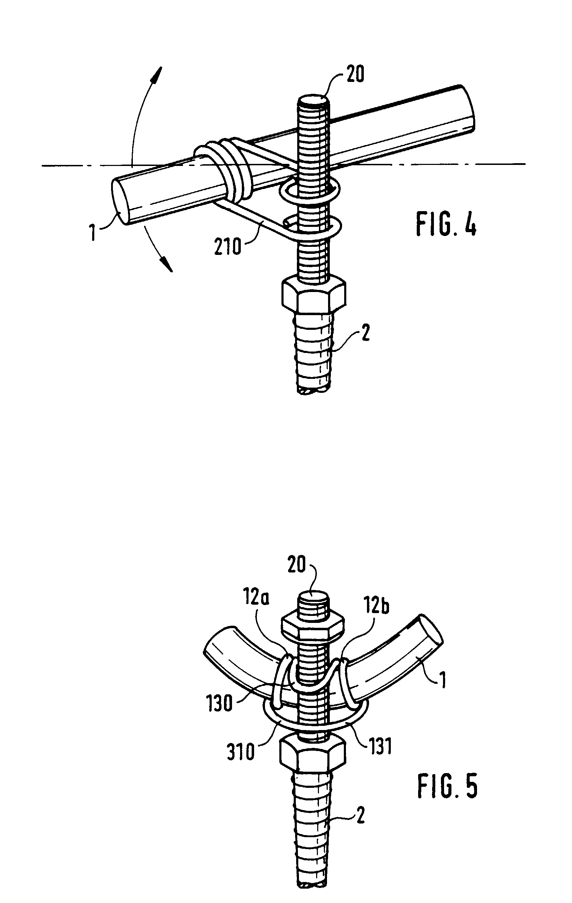

La figure 4 illustre les possibilités de souplesse angulaire permettant d'assembler un élément longitudinal 1 et un moyen d'ancrage 2 osseux non perpendiculaires entre eux.

Suivant le mode de réalisation de la Figure 5 l'enroulement en hélice 12a du brin 310 est d'au moins un tour dans un sens, puis forme une première demi boucle 230 destinée à recevoir la partie extra-osseuse 20 du moyen d'ancrage 2, avant de s'enrouler en hélice 12b de manière équivalente, en sens inverse autour de l'élément longitudinal 1 et de former une deuxième demi boucle 231 pour recevoir la partie extra-osseuse 20 du moyen d'ancrage 2.

La figure 5 illustre les possibilités de souplesse axiale permettant d'assembler un élément longitudinal 1 et un moyen d'ancrage 2 osseux, de manière à le bloquer en translation et en rotation même lorsque ledit élément 1 est déformé, cintré longitudinalement ou présente une surface irrégulière.

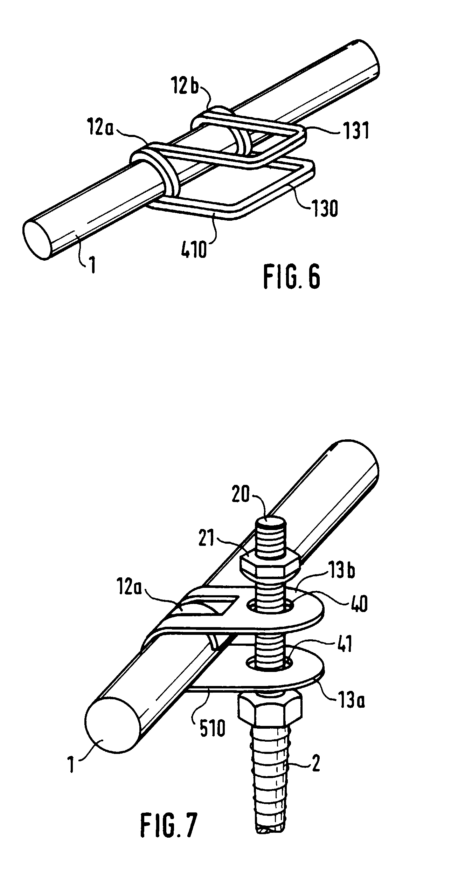

La figure 6 illustre un mode de réalisation de l'attache souple similaire à celui de la Figure 5, mais constituée d'un brin carré 410.

La figure 7 illustre un mode de réalisation de l'attache souple constituée d'un brin plat 510. Suivant ce mode de réalisation, le brin 510 est formé d'une bande métallique découpée de manière à former un enroulement 12a pour entourer l'élément longitudinal 1, et les extrémités 13a et 13b du brin 510 comportent chacunes un orifice 40 et 41 destiné à recevoir la partie extra-osseuse du moyen d'ancrage 2.

Dans tous les modes de réalisation, l'action de rapprochement par le moyen de serrage 21 réalise un déplacement angulaire des première et deuxième parties de connection du brin, ce qui réduit le diamètre intérieur du/ou des enroulement en hélices 12a et 12b de l'attache souple autour de l'élément longitudinal 1 de manière à le bloquer en translation et en rotation, même lorsque ledit élément 1 est déformé,cintré longitudinalement ou présente une surface irrégulière.

Bien que diverses modifications mineures puissent être suggérées par les hommes de métier spécialisés dans la présente technique, il est bien évident que d'autres formes de réalisation de la présente invention, à la portée de l'homme de métier, auraient également pu être envisagées sans pour cela sortir du cadre de celle-ci.

## Revendications

1. Dispositif d'attache souple destiné à solidariser un élément longitudinal (1) de forme essentiellement alongée et un moyen d'ancrage (2) osseux, interne notamment vertébral, ou externe, ledit moyen d'ancrage (2) comportant une partie intra-osseuse et une partie extra-osseuse (20) associée à un moyen de serrage (21),
ledit dispositif d'attache (10) comportant une première partie de connexion (13a) et une deuxième partie de connexion (13b) destinées à recevoir la partie extra-osseuse (20) du moyen d'ancrage (2), et
une partie intermédiaire (12a, 12b) réunissant lesdites première et deuxième parties de connexion (13a, 13b) destinée à enserrer l'élément longitudinal (1),
caractérisé en ce que le dispositif d'attache (10) souple est constitué par un brin (11) en matériau élastique ou encore à transformation de phase, et
en ce que ladite partie intermédiaire (12a, 12b) comprend au moins un enroulement en hélice (12a, 12b) adapté pour entourer l'élément longitudinal (1), de sorte qu'une action de rapprochement desdites première et deuxième parties de connexion (13a, 13b) par le moyen de serrage (21) ou par l'effet de transformation de phase du brin (11) en matériau à mémoire réduit le diamètre dudit enroulement en hélice (12a, 12b) de manière à bloquer l'élément longitudinal (1) en translation et en rotation.

2. Dispositif d'attache souple selon la revendication 1, caractérisé en ce que ledit brin (11) est de section circulaire, carrée ou rectangulaire comme un feuillard et est formé dans un matériau biocompatible de préférence métallique réalisant, par effet élastique ou mémoire, une position d'ouverture au repos permettant un coulissement dudit élément longitudinal (1).

3. Dispositif d'attache souple selon l'une des revendications 1 et 2, caractérisé en ce que ledit brin (11) réalise un premier enroulement en hélice (12a) d'au moins un tour dans un sens autour de l'élément longitudinal (1) à maintenir, puis réalise une boucle (13a) ou une demi boucle (13a) destinée à recevoir la partie extra-osseuse (20) du moyen d'ancrage (2), et puis réalise un second enroulement en hélice (12b) de préférence symétrique, en sens inverse autour de l'élément longitudinal (1) à maintenir.

4. Dispositif d'attache souple selon l'une des revendications 1 à 3, caractérisé en ce que les première et deuxième parties de connexion (13a, 13b) dudit brin (11) passent de part et d'autre du moyen d'ancrage (2) et sont formées chacunes par une boucle (13a, 13b), ou une demi-boucle (230, 231) autour de la partie extra osseuse (20) recevant le moyen de serrage (21) du moyen d'ancrage (2).

5. Dispositif d'attache souple selon l'une des revendications 1 à 4, caractérisé en ce qu'il comprend en outre une pièce coulissante (14), et en ce que les extrémités (30, 31) dudit brin (11) passent de part et d'autre du moyen d'ancrage (2) et sont réunies par ladite pièce coulissante (14) autour de la partie extra-osseuse (20) recevant le moyen de serrage (21) du moyen d'ancrage (2).

6. Dispositif d'attache souple selon la revendication 5, caractérisé en ce que la pièce coulissante (14) est positionnée le long des extrémités (30, 31) dudit brin (11) de manière à permettre un réglage de la distance entre l'élément longitudinal (1) et le moyen d'ancrage (2) osseux, puis se verrouille dans la position choisie par l'action du moyen de serrage (21).

7. Dispositif d'attache souple selon l'une des revendications 5 et 6, caractérisé en ce que la pièce coulissante (14) est munie d'un orifice (15) destiné à recevoir la partie extra-osseuse (20) et peut pivoter de selon 360 degrés en rotation, ou basculer ou se déplacer autour de l'axe longitudinal de ladite partie extra-osseuse (20).

8. Dispositif d'attache souple selon l'une des revendications 5 à 7, caractérisé en ce que l'orifice (15) de la pièce coulissante (14) destiné à recevoir la partie extra-osseuse (20) est en forme de sphère concave pour autoriser son réglage en bascule sur des embases sphériques du moyen de serrage (21) ou du moyen d'ancrage (2).

9. Dispositif d'attache souple selon la revendication 1, caractérisé en ce que la partie intermédiaire comporte un seul enroulement en hélice (12a) dont le nombre de tours est compris entre un et cinq, et en ce que une première extrémité dudit brin réalise une première boucle (230) et une seconde extrémité dudit brin réalise une seconde boucle (231) destinées à recevoir la partie extra-osseuse (20) du moyen d'ancrage (2), autour duquel elles peuvent pivoter, basculer ou coulisser pour permettre un assemblage où l'élément longitudinal (1) et le moyen d'ancrage (2) osseux sont non-perpendiculaires entre eux, ou un assemblage offrant plus de souplesse et moins d'alignement.

10. Dispositif d'attache souple selon la revendication 1, caractérisé en ce que ledit brin est constitué d'une bande métallique (510) découpée et adaptée pour être enroulée autour de l'élément longitudinal (1) et comportant deux orifices (40,41) destinés à recevoir la partie extra-osseuse du moyen d'ancrage (2).

11. Dispositif d'attache souple selon l'une des revendications 1 à 10, caractérisé en ce que la souplesse en torsion du ou des enroulements en hélice (12a, 12b) permet assembler un élément longitudinal (1) et un moyen d'ancrage (2) osseux non-perpendiculaires entre eux, ou un assemblage offrant plus de souplesse et moins de rappel par l'effet ressort du brin (11).

12. Dispositif d'attache souple selon l'une des revendications 1 à 11, caractérisé en ce que la souplesse axiale et radiale du ou des enroulements en hélice (12a, 12b) permettent de bloquer en translation et en rotation l'élément longitudinal (1), même lorsque ledit élément (1) est déformé, cintré longitudinalement, ou présente une surface irrégulière.

13. Dispositif d'attache souple selon l'une des revendications 1 à 12, caractérisé en ce que la souplesse axiale, radiale, et en torsion du ou des enroulements en hélice (12a, 12b) est choisie selon la forme ou la grosseur du brin (11).
